# EUROPÄISCHE PATENTSCHRIFT

(11) **EP 0 695 176 B1**
(45) Veröffentlichungstag und Bekanntmachung des Hinweises auf die Patenterteilung: **23.09.1998**
(21) Anmeldenummer: 94915104.7
(22) Anmeldetag: 23.04.1994
(51) Int. Cl.: A61K 9/50, A61K 31/34

(54) **FESTE DARREICHUNGSFORM VON ISOSORBID 5-MONONITRAT**
SOLID FORM OF ADMINISTRATION OF ISOSORBIDE 5-MONONITRATE
FORME SOLIDE D'ADMINISTRATION DE MONONITRATE-5 D'ISOSORBIDE

(30) Priorität: 27.04.1993 DE 4313726
(43) Veröffentlichungstag der Anmeldung: 07.02.1996
(73) Patentinhaber: Byk Gulden Lomberg Chemische Fabrik GmbH, 78467 Konstanz (DE); Byk Nederland BV, 1161 AB Zwanenburg (NL)
(72) Erfinder: VAN EGMOND, Cornelia Alida Maria, NL-2024 VP Haarlem (NL); PULLEN, André, NL-1661 BW Zwanenburg (NL); DE JONG, Sibo Wytse, NL-2024 TG Haarlem (NL); DE JONG, Adrianus Petrus, NL-1985 EW Driehuis (NL); BRON, Jan, NL-1871 EV Schoorl (NL)
(74) Vertreter: Wolf, Ulrich, Dr.
(86) Internationale Anmeldenummer: EP9401268
(87) Internationale Veröffentlichungsnummer: WO9425010

(56) Entgegenhaltungen:
- EP-A- 0 219 161
- EP-A- 0 263 083

## Beschreibung

Die Erfindung bezieht sich auf eine feste orale Darreichungsform für Isosorbid-5-mononitrat mit kontrollierter pH-unabhängiger Freigabe des Wirkstoffs im Magen-Darmtrakt in Form von mit einer Depotschicht und einer die Abgabe verlängernden Lackschicht überzogenen Pellets.

### Stand der Technik

Isosorbid-5-mononitrat findet aufgrund seiner blutgefäßdilatierenden Wirkung eine breite Anwendung in der Behandlung von cardiovaskulären Krankheiten. Ähnlich wie andere organische Nitrate, kann Isosorbid-5-mononitrat zu unerwünschten Nebenwirkungen wie insbesondere Kopfschmerzen, Erweiterung der Blutgefäße der Haut und Benommenheit führen. Diese Nebenwirkungen treten vor allem dann auf, wenn nach der Verabreichung die Blutspiegelwerte zu rasch ansteigen und/oder zu hohe Werte erreichen. Es hat daher nicht an Versuchen gefehlt, Darreichungsformen für Isosorbid-5-mononitrat zu schaffen, die den Wirkstoff verzögert über einen längeren Zeitraum abgeben.

In der EP-A 0163000 wird eine Darreichungsform für Isosorbid-5-mononitrat angegeben, bei der Kerne mit vier Schichten überzogen sind, nämlich von innen nach außen mit einer Isolierschicht, einer wirkstoffhaltigen Schicht, einer Deckschicht, sowie einer eine Anfangsdosis abgebenden Schicht. Das Produkt gibt eine Anfangsdosis frei und gibt den restlichen Wirkstoff anschließend über einen Zeitraum von 9 bis 10 Stunden ab. Diese vorteilhafte Abgabecharakteristik wird allerdings durch eine zeitaufwendige und sehr überwachungsintensive Herstellung erkauft.

In der EP-A 0202051 wird die Herstellung von Pellets enthaltenden Gelatinekapseln beschrieben. Die wirkstoffhaltigen Pellets werden durch Extrusion aus einem Gemisch von in nichtwässrigen Systemen quellbarer mikrokristalliner Cellulose und Wirkstoff hergestellt. Um das gewünschte Freigabeverhalten zu erreichen, ist es notwendig, dreierlei Pellets herzustellen. Es werden unbeschichtete Pellets, Pellets mit einer Schicht und Pellets mit zwei Schichten erzeugt und diese in einem bestimmten Verhaltnis in Kapseln abgefüllt. Zur Herstellung sind daher zahlreiche Arbeitsgänge erforderlich.

Eine weitere Darreichungsform basierend auf Pellets mit unterschiedlicher Abgabecharakteristik ist aus der EP-A 0396425 bekannt. In der EP-A 0219161 wird die Herstellung von Isosorbit-5-mononitrat enthaltenden Tabletten offenbart, die zur Erhöhung der Stabilität Polyvinylpyrrolidon enthalten. In der WO-A 92/01446 werden Granulate für leichtlösliche Wirkstoffe beschrieben, die ein Subcoating aus Wachs oder wachsartigen Polymeren zur Vermeidung des Wanderns des Wirkstoffs nach außen aufweisen. In der EP-A-0263083 werden Überzugsmembrane für u. a. Isosorbid-5-mononitrat offenbart, die einerseits aus Fettsäuren und/oder Paraffin und andererseits härtenden Substanzen, wie u. a. Ethylcellulose und Polymethacrylat zusammengesetzt sind.

### Beschreibung der Erfindung

Eine Aufgabe der vorliegenden Erfindung war die Zurverfügungstellung einer festen oralen Darreichungsform für Isosorbid-5-mononitrat mit kontrollierter, pH-unabhängiger Freigabecharakteristik, die bezüglich der Bioäquivalenz den bereits auf dem Markt befindlichen Darreichungsformen zumindest ebenbürtig ist, sich jedoch mit geringerem Aufwand in großen Chargen in einem voll automatisierbaren Prozeß fertigen läßt, d. h. die Zahl der unterschiedlichen Herstellungsschritte sollte im Vergleich zu den vorbekannten Verfahren möglichst erniedrigt werden.

Diese Aufgabe wird erfindungsgemäß durch eine Darreichungsform gelöst, bei der die Lackschicht Ethylcellulose und Polymethacrylat, e.g. Eudragit® RS-100 im Gewichtsverhältnis 3:1 bis 3:2 sowie einen Weichmacher enthält. Gegenstand der Erfindung ist daher eine feste orale Darreichungsform für Isosorbid-5-mononitrat mit kontrollierter, pH-unabhängiger Freigabe des Wirkstoffs im Magen-Darmtrakt in Form von mit einer Depotschicht und einer die Abgabe verlängernden Lackschicht überzogenen Pellets, dadurch gekennzeichnet, daß die Lackschicht Ethylcellulose und Polymethacrylat, e.g. Eudragit® RS-100 im Gewichtsverhältnis von 3:1 bis 3:2 sowie einen Weichmacher enthält.

Weitere Gegenstände ergeben sich aus den Ansprüchen.

Besonders bevorzugt ist ein Gewichtsverhältnis von Ethylcellulose und Polymethacrylat, e.g. Eudragit® RS-100 von 2:1. Als Weichmacher für die Lackschicht eignen sich die üblicherweise für solche Zwecke verwendeten Weichmacher. Besonders bevorzugt ist die Verwendung von Dibutylphthalat. In der Depotschicht sind Hydroxypropylmethylcellulose und Polyvinylpyrrolidon in einem Gewichtsverhältnis von 2:1 bis 1:2, vorzugsweise etwa 1:1 enthalten. Es ist vorteilhaft, bei der Herstellung der Depotschicht zusätzlich Polyethylenglykol einzusetzen.

Das Aufbringen von Wirkstoffen in einer Depotschicht auf inerte Zuckerkügelchen durch Besprühen mit einer organischen und/oder wässrigen Lösung oder einer Suspension ist dem Fachmann bekannt. Bei Anwendung des bekannten Verfahrens auf Isosorbid-5-mononitrat beobachtet man entweder bereits nach dem Trocknen der Depotschicht oder erst nach Lagerung über eine gewisse Zeit, daß die Pellets eine unregelmäßige Oberfläche aufweisen. Dies wird darauf zurückgeführt, daß Isosorbid-5-mononitrat zum Auskristallisieren aus der Beschichtungslösung bzw. -suspension neigt. Dieses Auskristallisieren führt zu einem nicht mehr steuerbaren Freigabeverhalten. Es wurde nun gefunden, daß dieses Problem überraschend einfach gelöst werden kann, indem man die gesamte Menge an Isosorbid-5-mononitrat in einem Bruchteil, beispielsweise der Hälfte der Beschichtungslösung bzw. -suspension aufträgt, unmittelbar gefolgt vom Rest der wirkstofffreien Lösung bzw. Suspension. In einer bevorzugten Ausführungsform des erfindungsgemäßen Verfahrens wird eine Mischung von Ethanol, entmineralisiertem Wasser, Hydroxypropylmethylcellulose, Polyvinylpyrrolidon, Talkum und Polyethylenglykol hergestellt. In etwa 45% dieser Mischung wird die gesamte aufzutragende Menge Isosorbid-5-mononitrat gelöst. Anschließend werden die inerten Zuckerkügelchen in einem Verfahrensschritt mit dem wirkstoffhaltigen und dem wirkstofffreien Teil der Suspension beschichtet. Die erhaltenen Pellets geben den Wirkstoff vollständig und annähernd augenblicklich sowohl beim pH-Wert des Magens (pH 1,2) als auch bei dem des Duodenum (pH 7,4) ab. Die wirkstoffhaltigen Pellets werden sodann mit einer Schicht versehen, die zu einer pH-unabhängigen, kontrollierten Freigabe führt. Üblicherweise werden nach dem Stand der Technik bei derartigen Problemstellungen Überzüge aus Ethylcellulose angewendet. Im vorliegenden Fall konnte zwar durch Überzüge aus Ethylcellulose eine pH-unabhängige Freigabe erreicht werden. Jedoch war die Freigabe zu rasch. Um das gewünschte Freigabeprofil zu erhalten, wäre eine unverhältnismäßig große Menge von Ethylcellulose erforderlich gewesen, was zu einer unwirtschaftlichen Verlängerung des Herstellungsverfahrens führen würde. Auch Versuche mit Überzügen aus verschiedenen Typen von Polymethacrylaten, die nach einschlägigen Publikationen und Produktinformationen des Herstellers zur Herstellung von pH-unabhängig freigebenden Überzügen geeignet sind, ergaben nicht das gewünschte Freigabeprofil. Überraschenderweise lassen sich jedoch aus Beschichtungslösungen, die sowohl Ethylcellulose als auch Polymethacrylat zusammen mit einem Weichmacher enthalten, Überzugsmembranen erzeugen, die reproduzierbar zu einem pH-unabhängigen Freigabeprofil führen. Als Ethylcellulose besonders geeignet ist Ethocel® 10 cps (Dow Chemical Company). Als Polymethacrylat hat sich insbesondere Eudragit® RS-100 (Röhm Pharma GmbH) als geeignet erwiesen. Das Gewichtsverhältnis von Ethylcellulose und Polymethacrylat in der Beschichtungslösung bzw. -suspension liegt vorteilhaft zwischen 3:1 und 3:2. Besonders bevorzugt ist ein Gewichtsverhältnis von 2:1. Als Weichmacher ist Dibutylphthalat bevorzugt. Die Beschichtungskomponenten werden vorzugsweise gelöst in Aceton in Gegenwart von entmineralisiertem Wasser aufgebracht. Sowohl für die Aufbringung der Depotschicht als auch der das Abgabeverhalten steuernden Schicht bestehen keine besonderen apparativen Anforderungen. Das Coating kann in den für solche Zwecke üblicherweise verwendeten Beschichtungsapparaten erfolgen.

### Herstellungsbeispiel

Für die Erzeugung einer Depotschicht wird folgende Mischung hergestellt..

| | |
|---|---|
| Ethanol | 7899 ml |
| entmineralisiertes Wasser | 878 ml |
| Hydroxypropylmethylcellulose (Pharmacoat® 3 cps) | 438,9 g |
| Polyvinylpyrrolidon (Kollidone® 25) | 438,9 g |
| Talkum | 131,7 g |
| Polyethylenglykol (PEG 6000) | 43,9 g |

In ungefähr 45 Teilen dieser Suspension wurden 787,8 g Isosorbid-5-mononitrat gelöst. Diese konzentrierte Suspension, die ungefähr 197,0 g Polyvinylpyrrolidon, 197,0 g Hydroxypropylmethylcellulose, 59,1 g Talkum und 19,7 g Polyethylenglykol enthält, wird auf 1158,6 g inerte Zuckerkügelchen von 0,5 bis 0,6 mm Durchmesser gesprüht. Dies kann in den üblichen Apparaturen, wie beispielsweise einem Wirbelschichtcoater mit Trockner, der vorteilhafterweise einen Rotor enthält, geschehen. Unmittelbar anschließend werden die restlichen 55 Teile der obengenannten wirkstofffreien Suspension, die ungefähr 241,9 g Hydroxypropylmethylcellulose, 241,9 g Polyvinylpyrrolidon, 72,6 g Talkum und 24,2 g Polyethylenglykol enthält, aufgetragen. Die erhaltenen Pellets weisen eine mikroskopisch glatte Oberfläche auf und geben Isosorbid-5-monohydrat rasch frei.

Diese Pellets werden zur Erzeugung einer retardierenden Lackschicht mit folgender Lösung gecoatet:

| | |
|---|---|
| Aceton | 497,0 g |
| entmineralisiertes Wasser | 33,3 g |
| Ethylcellulose (Ethocel® 10 cps) | 33,3 g |
| Polymethacrylat (Eudragit® RS-100) | 16,7 g |
| Dibutylphthalat | 5,0 g |
| Talkum | 0,5 g |

Die erhaltenen Pellets zeigen eine pH-unabhängige Freigabe von Isosorbid-5-mononitrat, die der Freigabe der bewährten Produkte entspricht. Versuche an freiwilligen Probanden ergaben, daß die erfindungsgemäßen Pellets mit den bewährten Produkten bioäquivalent sind.

Es steht somit ein den bewährten Zubereitungen ebenbürtiges Produkt zur Verfügung, das mit erheblich geringerem Aufwand hergestellt werden kann als diese.

## Patentansprüche

1. Feste orale Darreichungsform für Isosorbid-5-mononitrat mit kontrollierter, pH-unabhängiger Freigabe des Wirkstoffs im Magen-Darmtrakt in Form von mit einer Depotschicht und einer die Abgabe verlängernden Lackschicht überzogenen Pellets, dadurch gekennzeichnet, daß die Lackschicht Ethylcellulose und Polymethacrylat, e.g. Eudragit® RS-100, im Gewichtverhältnis von 3:1 bis 3:2 sowie einen Weichmacher enthält.

2. Darreichungsform nach Anspruch 1, dadurch gekennzeichnet, daß die Lackschicht Ethylcellulose und Polymethacrylat, e.g. Eudragit® RS-100, im Gewichtverhältnis von 2:1 enthält.

3. Darreichungsform nach Anspruch 1, dadurch gekennzeichnet, daß die Lackschicht als Weichmacher Dibutylphthalat enthält.

4. Darreichungsform nach Anspruch 1, dadurch gekennzeichnet, daß die Depotschicht Hydroxypropylmethylcellulose und Polyvinylpyrrolidon im Gewichtsverhältnis von 2:1 bis 1:2 enthält.

5. Darreichungsform nach Anspruch 4, dadurch gekennzeichnet, daß die Depotschicht zusätzlich Polyethylenglykol enthält.

6. Darreichungsform nach Anspruch 4, dadurch gekennzeichnet, daß sich der Wirkstoff überwiegend auf der kernnahen Seite der Depotschicht befindet.

7. Verfahren zur Herstellung einer festen oralen Darreichungsform für Isosorbid-5-mononitrat mit kontrollierter, pH-unabhängiger Freigabe des Wirkstoffs im Magen-Darmtrakt in Form von mit einer Depotschicht und einer die Abgabe verlängernden Lackschicht überzogenen Pellets, dadurch gekennzeichnet, daß man auf inerte Zuckerkügelchen in einem Arbeitsgang eine Lösung von Isosorbid-5-mononitrat in einem Teil in einer wässrigethanolischen Suspension enthaltend Hydroxypropylmethylcellulose und Polyvinylpyrrolidon und dann den Rest der gesamten Suspension ohne Wirkstoff aufsprüht und die erhaltenen wirkstoffhaltigen Pellets mit einer Ethylcellulose und Polymethacrylat, e.g. Eudragit® RS-100, im Gewichtsverhältnis von 3:1 bis 3:2 sowie einen Weichmacher enthaltenden Lösung besprüht.

## Claims

1. Solid oral administration form for isosorbide-5-mononitrate with controlled, pH-independent release of the active compound in the gastrointestinal tract in the form of pellets coated with a depot layer and a coating layer prolonging release, characterized in that the coating layer contains ethylcellulose and polymethacrylate, e.g. Eudragit® RS-100, in the weight ratio from 3:1 to 3:2, and a plasticizer.

2. Administration form according to Claim 1, characterized in that the coating layer contains ethylcellulose and polymethacrylate, e.g. Eudragit® RS-100, in the weight ratio of 2:1.

3. Administration form according to Claim 1, characterized in that the coating layer contains dibutyl phthalate as plasticizer.

4. Administration form according to Claim 1, characterized in that the depot layer contains hydroxypropylmethylcellulose and polyvinylpyrrolidone in the weight ratio from 2:1 to 1:2.

5. Administration form according to Claim 4, characterized in that the depot layer additionally contains polyethylene glycol.

6. Administration form according to Claim 4, characterized in that the active compound is mainly situated on the side of the depot layer near to the core.

7. Process for the production of a solid oral administration form for isosorbide-5-mononitrate with controlled, pH-independent release of the active compound in the gastrointestinal tract in the form of pellets coated with a depot layer and a coating layer prolonging release, characterized in that a solution of isosorbide-5-mononitrate in one part in an aqueous-ethanolic suspension containing hydroxypropylmethylcellulose and polyvinylpyrrolidone is sprayed onto inert sugar globules in one operation and the rest of the total suspension without active compound is sprayed on and the active compound-containing pellets obtained are sprayed with a solution containing ethylcellulose and polymethacrylate, e.g. Eudragit® RS-100, in the weight ratio from 3:1 to 3:2, and a plasticizer.

## Revendications

1. Forme solide d'administration par voie orale de mononitrate-5 d'isosorbide permettant une libération contrôlée, indépendante du pH, du principe actif dans le tractus intestinal et qui se présente sous forme de pellets enrobés d'une couche de dépôt et d'une couche de vernis prolongeant la libération, laquelle forme d'administration est caractérisée en ce que la couche de vernis contient de l'éthylcellulose et du polyméthacrylate, par exemple de l'Eudradit® RS-100, en un rapport en poids allant de 3/1 à 3/2 en conjonction avec un agent plastifiant.

2. Forme d'administration selon la revendication 1, caractérisée en ce que la couche de vernis contient de l'éthylcellulose et du polyméthacrylate, par exemple de l'Eudradit® RS-100, en un rapport en poids égal à 2/1.

3. Forme d'administration selon la revendication 1, caractérisée en ce que la couche de vernis contient comme agent plastifiant du phtalate de dibutyle.

4. Forme d'administration selon la revendication 1, caractérisée en ce que la couche de dépôt contient de l'hydroxypropylméthylcellulose et de la polyvinylpyrrolidone en un rapport en poids allant de 2/1 à 1/2.

5. Forme d'administration selon la revendication 4, caractérisée en ce que la couche de dépôt contient en outre du polyéthylèneglycol.

6. Forme d'administration selon la revendication 4, caractérisée en ce que le principe actif se trouve majoritairement dans la partie proche du noyau de la couche de dépôt.

7. Procédé de préparation d'une forme solide d'administration par voie orale de mononitrate-5 d'isosorbide permettant une libération contrôlée, indépendante du pH, du principe actif dans le tractus intestinal et qui se présente sous forme de pellets enrobés d'une couche de dépôt et d'une couche de vernis prolongeant la libération, lequel procédé est caractérisé en ce que l'on vaporise, sur les billes de sucre inertes, en une seule opération, une solution de mononitrate-5 d'isosorbide dans une fraction d'une suspension hydroéthanolique contenant de l'hydroxypropylméthylcellulose et de la polyvinylpyrrolidone et en ce que l'on applique ensuite le reste de la suspension exempte de principe actif, et en ce que l'on vaporise sur les pellets contenant le principe actif ainsi obtenus, une solution contenant de l'éthylcellulose et du polyméthacrylate, par exemple de l'Eudragit® RS-100, en un rapport en poids allant de 3/1 à 3/2 ainsi qu'un agent plastifiant.
